# EUROPÄISCHE PATENTSCHRIFT

(11) **EP 1 414 567 B1**
(45) Veröffentlichungstag und Bekanntmachung des Hinweises auf die Patenterteilung: **18.06.2014**
(21) Anmeldenummer: 02791454.8
(22) Anmeldetag: 16.07.2002
(51) Int. Cl.: B01J 31/18, B01J 31/24, C07C 253/10

(54) **NI(0) ENTHALTENDES KATALYSATORSYSTEM FÜR HYDROCYANIERUNG**
CATALYST SYSTEM CONTAINING NI(0) FOR HYDROCYANATION
SYSTEME CATALYSEUR CONTENANT NI(0) POUR HYDROCYANATION

(30) Priorität: 27.07.2001 DE 10136488
(43) Veröffentlichungstag der Anmeldung: 06.05.2004
(73) Patentinhaber: BASF SE, 67056 Ludwigshafen (DE)
(72) Erfinder: BARTSCH, Michael, 67433 Neustadt (DE); BAUMANN, Robert, 68161 Mannheim (DE); KUNSMANN-KEITEL, Dagmar, Pascale, 67117 Limburgerhof (DE); HADERLEIN, Gerd, 67269 Grünstadt (DE); JUNGKAMP, Tim, 2950 Kapellen (BE); ALTMAYER, Marco, 68199 Ilvesheim (DE); SIEGEL, Wolfgang, 67117 Limburgerhof (DE)
(86) Internationale Anmeldenummer: PCT/EP2002/007888
(87) Internationale Veröffentlichungsnummer: WO 2003/011457

(56) Entgegenhaltungen:
- WO-A-99/13983
- WO-A-99/64155
- WO-A1-99/06357
- US-A- 3 496 215
- US-A- 5 512 696
- US-A- 5 696 280

## Beschreibung

Die vorliegende Erfindung betrifft als Katalysator geeignetes System enthaltend
a) Ni(0)
b) 4 bis 10 mol pro mol Ni(0) gemäß a) eine Verbindung (I) der Formel

   P(X¹R¹)(X²R²)(X³R³) (I)

   mit
   - X¹, X², X³: Sauerstoff
   - R¹, R², R³: unabhängig voneinander gleiche oder unterschiedliche organische Reste
   und
c) 1 bis 4 mol pro mol Ni(0) gemäß a) eine Verbindung (II) der Formel 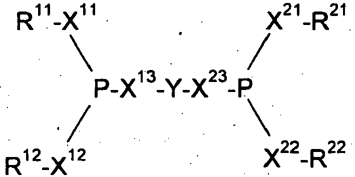 mit
   - X¹¹, X¹², X¹³ X²¹, X²², X²³: Sauerstoff
   - R¹¹, R¹²: unabhängig voneinander gleiche oder unterschiedliche, einzelne oder verbrückte organische Reste
   - R²¹, R²²: unabhängig voneinander gleiche oder unterschiedliche, einzelne oder verbrückte organische Reste
   - Y: Brückengruppe
   - R¹, R², R³: unabhängig voneinander Phenyl-, o-Tolyl-, m-Tolyl-, oder p-Tolyl-Gruppe
sowie Verfahren zur Herstellung solcher Systeme.

Als Katalysator für die Hydrocyanierung von Butadien zu einem Gemisch isomerer Pentennitrile und von Pentennitril zu Adipodinitril geeignete Systeme aus Ni(0) und einer Verbindung (II) und Verfahren zu ihrer Herstellung sind an sich bekannt, beispielsweise aus US 3,903,120, US 5,523,453, US 5,981,772, US 6,127,567, US 5,693,843, US 5,847,191, WO 01/14392.

Ferner sind aus WO-A-99/64155 und WO-A-99/13983 Katalysatoren für die Herstellung von Nitrilen bekannt, die wenigstens einen Komplex eines Metalls der VIII. Nebengruppe mit mindestens einem zweizähnigen Phosphonitliganden umfassen.

Die Herstellung dieser Katalysatorsysteme ist technisch aufwendig und teuer. Dies gilt insbesondere, als dass die Katalysatorsysteme im Laufe ihrer Verwendung allmählich zersetzt werden und somit ausgeschleust und durch neuen Katalysator ersetzt werden müssen.

Die direkte Umsetzung von metallischem Nickel als Ni(0)-Quelle mit Verbindung (II) mit oder ohne flüssigem Verdünnungsmittel oder Halogenwasserstoff als Katalysator führt in hohem Umfang zu einer Zersetzung von Verbindung (II).

Die Verwendung von Ni-bis-1,4-cyclooctadien als Ni(0)'-haltige Ausgangsverbindung ermöglicht zwar die Herstellung des Systems aus Ni(0) und Verbindung (II). Nachteilig bei diesem Verfahren ist jedoch die aufwendige und teure Herstellung, des Ni-bis-1,4-cyclooctadiens.

Gleiches gilt für die Verwendung von Ni(p(O-o-C₆H₄CH₃)₃)₂(C₂H₄) als Ni(0)-haltige Ausgangsverbindung.

Bekannt ist die Herstellung des Systems aus Ni(0) und Verbindung (II) ausgehend von Nickelchlorid und Zink als Ni(0)-Quelle. Nachteilig bei diesem Verfahren ist die gleichzeitige Bildung des genannten Katalysatorsystems und Zinkchlorid.

Ist die Verwendung des reinen Katalysatorsystems beabsichtigt, so muss vor der Verwendung zunächst das Zinkchlorid aufwendig abgetrennt werden.

Setzt man statt des reinen Katalysatorsystems das Gemisch aus Katalysatorsystem und Zinkchlorid ein, so gestaltet sich die Aufarbeitung des Gemischs aus verbrauchtem, ausgeschleustem Katalysatorsystem und Zinkchlorid als außerordentlich problematisch.

Ein weiterer Nachteil der Katalysatorsysteme aus Ni(0) und Verbindung (II) ist, dass Verbindung (II) nur durch eine technisch aufwendige und teure Synthese erhalten werden kann.

Der vorliegenden Erfindung lag die Aufgabe zugrunde, ein Katalysatorsystem bereitzustellen, das auf technisch einfache und wirtschaftliche Weise synthetisiert werden kann und das, insbesondere bei der Hydrocyanierung von Verbindungen mit konjugierten olefinischen Doppelbindungen, wie Butadien, als auch Verbindungen mit einer olefinischen Doppelbindung und einer anderen ungesättigten Gruppe, wie 2-Pentennitril, 3-Pentennitril, 4-Pentennitril, 2-Pentensäureester, 3-Pentensäureester oder 4-Pentensäureester, gegenüber einem Katalysatorsystem aus Ni(0) und Verbindung (II) vergleichbare Selektivitäten und Aktivitäten aufweist.

Demgemäß wurde das eingangs definierte System sowie ein Verfahren zu seiner Herstellung gefunden.

Erfindungsgemäß setzt man als Verbindung a) Ni(0) ein.

Vorteilhaft kommt der Einsatz von metallischem Nickel als Ni(0) in Betracht, wobei dem metallischem Nickel weitere Elemente zulegiert sein können. In einer bevorzugten Ausführungsform kann man reines metallisches Nickel einsetzen. Im Sinne der vorliegenden Erfindung kann das reine metallische Nickel die in kommerzieller Ware an sich üblichen Verunreinigungen enthalten.

Die geometrische Form des metallischen Nickels ist an sich nicht kritisch. Es hat sich allerdings als vorteilhaft erwiesen, zur Erzielung einer hohen Umsetzungsgeschwindigkeit in Schritt a) des erfindungsgemäßen Verfahrens metallisches Nickel mit einer groβen Oberfläche pro Gewichtseinheit einzusetzen. Als solches kommen beispielsweise Nickelschwamm oder vorzugsweise feinteiliges Nickelpulver in Betracht. Derartiges großoberflächiges metallisches Nickel ist an sich bekannt und kommerziell erhältlich. Erfindungsgemäß weist Verbindung (I) die Formel

P(X¹R¹)(X²R²)(X³R³) (I)

auf.

Unter Verbindung (I) wird im Sinne der vorliegenden Ereindung eine einzelne Verbindung oder ein Gemisch verschiedener Verbindungen der vorgenannten Formel verstanden.

Erfindungsgemäß stehen alle der Gruppen X¹, X² und X³ für Sauerstoff, so dass Verbindung (I) ein Phosphit der Formel P(OR¹)(OR²)(OR³) mit den für R¹, R² und R³ in dieser Beschreibung genannten Bedeutungen darstellt.

Erfindungsgemäß stehen R¹, R², R³ unabhängig voneinander für gleiche oder unterschiedliche organische Reste.

Erfindungsgemäß sind die Gruppen R¹, R² und R³ Reste ausgewählt aus der Gruppe bestehend aus Phenyl, o-Tolyl, m-Tolyl und p-Tolyl.

In einer besonders bevorzugten Ausführungsform sollten dabei maximal zwei der Gruppen R¹, R² und R³ Phenyl-Gruppen sein.

In einer anderen bevorzugten Ausführungsform sollten dabei maximal zwei der Gruppen R¹, R² und R³ o-Tolyl-Gruppen sein.

Als besonders bevorzugte Verbindungen (I) können solche der Formel (o-Tolyl-O-)_{w} (m-Tolyl-O-)ₓ (p-Tolyl-O-)_{y} (Phenyl-O-)_{z} P
mit w, x, y, z eine natürliche Zahl
mit w + x + y + z = 3 und
w, z kleiner gleich 2'
eingesetzt werden, wie (p-Tolyl-O-)(Phenyl)₂P, (m-Tolyl-O-)(Phenyl)₂P, (o-Tolyl-O-)(Phenyl)₂P, (p-Tolyl-O-)₂(Phenyl)P, (m-Tolyl-O-)₂(Phenyl)P, (o-Tolyl-O-)₂(Phenyl)P, (m-Tolyl-O-)(p-Tolyl-O-)(Phenyl)P, (o-Tolyl-O-)(p-Tolyl-O-)(Phenyl)P, (o-Tolyl-O-)(m-Tolyl-O-)(Phenyl)P, (p-Tolyl-O-)₃P, (m-Tolyl-O-)(p-Tolyl-O-)₂P, (o-Tolyl-O-)(p-Tolyl-O-)₂P, (m-Tolyl-O-)₂(p-Tolyl-O-)P, (o-Tolyl-O-)₂(p-Tolyl-O-)P, (o-Tolyl-O-)(m-Tolyl-O-)(p-Tolyl-O-)P, (m-Tolyl-O-)₃P, (o-Tolyl-O-)(m-Tolyl-O-)₂P (o-Tolyl-O-)₂(m-Tolyl-O-)P oder Gemische solcher Verbindungen eingesetzt werden.

So können beispielsweise Gemische enthaltend (m-Tolyl-O-)₃P, (m-Tolyl-O-)₂(p-Tolyl- - O-)P, (m-Tolyl-O-)(p-Tolyl-O-)₂P und (p-Tolyl-O-)₃P durch Umsetzung eines Gemisches enthaltend m-Kresol und p-Kresol, insbesondere im Molverhältnis 2:1, wie es bei der destillativen Aufarbeitung von Erdöl anfällt, mit einem Phosphortrihalogenid, wie Phosphortrichlorid, erhalten werden.

Solche Verbindungen (I) und deren Herstellung sind an sich bekannt.

Erfindungsgemäß weist das System ein Verhältnis im Bereich von 4 bis 10 mol, vorzugsweise 4 bis 8 mol, insbesondere 4 bis 6 mol, Verbindung (I) pro mol Ni(0) auf.

Erfindungsgemäß weist Verbindung (II) die Formel 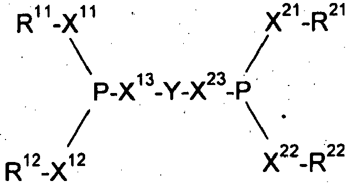 mit
- X¹¹, X¹², X¹³ X²¹, X²², X²³: Sauerstoff
- R¹¹, R¹²: unabhängig voneinander gleiche oder unterschiedliche, einzelne oder verbrückte organische Reste
- R²¹, R²²: unabhängig voneinander gleiche oder unterschiedliche, einzelne oder verbrückte organische Reste,
- Y: Brückengruppe
auf.

Unter Verbindung (II) wird im Sinne der vorliegenden Erfindung eine einzelne Verbindung oder ein Gemisch verschiedener Verbindungen der vorgenannten Formel verstanden.

Als Brückengruppe Y kommen vorteilhaft substituierte, beispielsweise mit C₁-C₄-Alkyl, Halogen, wie Fluor, Chlor. Brom, halogeniertem Alkyl, wie Trifluormethyl, Aryl, wie Phenyl, oder unsubstituierte, Arylgruppen in Betracht, vorzugsweise solche mit 6 bis 20 Kohlenstoffatomen im aromatischen System, insbesondere Pyrocatechol, Bis(phenol) oder Bis(naphthol).

Die Reste R¹¹ und R¹² können unabhängig voneinander gleiche oder unterschiedliche organische Reste darstellen. Vorteilhaft kommen als Reste R¹¹ und R¹² Arylreste, vorzugsweise solche mit 6 bis 10 Kohlenstoffatomen, in Betracht, die unsubstituiert oder einfach oder mehrfach substituiert sein können, insbesondere durch C1-C4-Alkyl, Halogen, wie Fluor, Chlor. Brom, halogeniertem Alkyl, wie Trifluormethyl, Aryl, wie Phenyl, oder unsubstituierte Arylgruppen.

Die Reste R²¹ und R²² können unabhängig voneinander gleiche oder unterschiedliche organische Reste darstellen. Vorteilhaft kommen als Reste R²¹ und R²² Arylreste, vorzugsweise solche mit 6 bis 10 Kohlenstoffatomen, in Betracht, die unsubstituiert oder einfach oder mehrfach substituiert sein können, insbesondere durch C1-C4-Alkyl, Halogen, wie Fluor, Chlor. Brom, halogeniertem Alkyl, wie Trifluormethyl, Aryl, wie Phenyl, oder unsubstituierte Arylgruppen.

Die Reste R¹¹ und R¹² können einzeln oder verbrückt sein.

Die Reste R²¹ und R²² können einzeln oder verbrückt sein.

Die Reste R¹¹, R¹², R²¹ und R²² können alle einzeln, zwei verbrückt und zwei einzeln oder alle vier verbrückt sein in der beschriebenen Art.

In einer besonders bevorzugten Ausführungsform kommen die in US 5,723,641 genannten Verbindungen der Formel I, II, III, IV und V in Betracht.

In einer besonders bevorzugten Ausführungsform kommen die in US 5,512,696 genannten Verbindungen der Formel I, II, III, IV, V, VI und VII, insbesondere die dort in den Beispielen 1 bis 31 eingesetzte Verbindungen, in Betracht.

In einer besonders bevorzugten Ausführungsform kommen die in US 5,821,378 genannten Verbindungen der Formel I, II, III, IV, V, VI, VII, VIII, IX, X, XI, XII, XIII, XIV und XV, insbesondere die dort in den Beispielen 1 bis 73 eingesetzte Verbindungen, in Betracht.

In einer besonders bevorzugten Ausführungsform kommen die in US 5,512,695 genannten Verbindungen der Formel I, II, III, IV, V und VI, insbesondere die dort in den Beispielen 1 bis 6 eingesetzte Verbindungen, in Betracht.

In einer besonders bevorzugten Ausführungsform kommen die in US 5,981,772 genannten Verbindungen der Formel I, II, III, IV, V, VI, VII, VIII, IX, X, XI, XII, XIII und XIV, insbesondere die dort in den Beispielen 1 bis 66 eingesetzte Verbindungen, in Betracht.

In einer besonders bevorzugten Ausführungsform kommen die in US 6,127,567 genannten Verbindungen und dort in den Beispielen 1 bis 29 eingesetzte Verbindungen in Betracht.

In einer besonders bevorzugten Ausführungsform kommen die in US 6,020,516 genannten Verbindungen der Formel I, II, III, IV, V, VI, VII, VIII, IX und X, insbesondere die dort in den Beispielen 1 bis 33 eingesetzte Verbindungen, in Betracht.

In einer besonders bevorzugten Ausführungsform kommen die in US 5,959,135 genannten Verbindungen und dort in den Beispielen 1 bis 13 eingesetzte Verbindungen in Betracht.

In einer besonders bevorzugten Ausführungsform kommen die in US 5,847,191 genannten Verbindungen der Formel I, II and III in Betracht.

In einer besonders bevorzugten Ausführungsform kommen die in US 5,523,453 genannten Verbindungen, insbesondere die dort in Formel 1, 2, 3, 4, 5, 6, 7, 8, 9, 10, 11, 12, 12, 13, 14, 15, 16, 17, 18, 19, 20 und 21 dargestellten Verbindungen, in Betracht.

In einer besonders bevorzugten Ausführungsform kommen die in WO 01/14392 genannten Verbindungen, vorzugsweise die dort in Formel V, VI, VII, VIII, IX, X, XI, XII, XIII, XIV, XV, XVI, XVII, XXI, XXII, XXIII dargestellten Verbindungen, in Betracht.

In einer besonders bevorzugten Ausführungsform kommen die in WO 98/27054 genannten Verbindungen in Betracht.

Solche Verbindungen (II) und deren Herstellung sind an sich bekannt.

Erfindungsgemäß weist das System ein Verhältnis im Bereich von 1 bis 4 mol, vorzugsweise 1 bis 3 mol Verbindung (II) pro mol Ni(0) auf, wobei insbesondere im Falle von schwierig herstellbaren oder teuren Verbindungen (II) in einer besonderen Ausführungsform ein Verhältnis im Bereich von 1 bis 2 mol Verbindung (II) zu Ni(0) in Betracht kommen kann.

Vorteilhafter Weise sollten Verbindung (I) und Verbindung (II) fähig sein, mit Ni(0) Komplexverbindungen zu bilden. Verbindung (I) weist im allgemeinen eine zur Bindung zu Ni(0) fähige Koordinationsstelle auf, während Verbindung (II) je nach Geometrie, Bindungsstärke und der Gegenwart anderer auf Ni(0) koordinierend wirkender Verbindungen, wie Verbindung (I), im allgemeinen eine oder zwei zur Bindung zu Ni(0) fähige Koordinationsstellen aufweist.

In einer bevorzugten Ausführungsform enthält das erfindungsgemäße System einen Ni(0)-Komplex der Formel Ni(0) (Verbindung (I))ₓ (Verbindung(II)) mit x = 1, 2.

Erfindungsgemäß können die Systeme erhalten werden, indem man
a) Ni(0) mit einer Verbindung (I) in Gegenwart eines flüssigen Verdünnungsmittels umsetzt unter Erhalt eines ersten Systems enthaltend Ni(0) und Verbindung (I) und dann
b) dieses erste System mit einer Verbindung (II) in Gegenwart eines flüssigen Verdünnungsmittels umsetzt unter Erhalt eines erfindungsgemäßen Systems.

Erfindungsgemäß setzt man in Schritt a) Ni(0) ein.

Vorteilhaft kommt der Einsatz von metallischem Nickel als Ni(0) in Betracht, wobei dem metallischem Nickel weitere Elemente zulegiert sein können. In einer bevorzugten Ausführungsform kann man reines metallisches Nickel einsetzen. Im Sinne der vorliegenden Erfindung kann das reine metallische Nickel die in kommerzielle Ware an sich üblichen Verunreinigungen enthalten.

Die geometrische Form des metallischen Nickels ist an sich nicht kritisch. Es hat sich allerdings als vorteilhaft erwiesen, zur Erzielung einer hohen Umsetzungsgeschwindigkeit in Schritt a) des erfindungsgemäßen Verfahrens metallisches Nickel mit einer großen Oberfläche pro Gewichtseinheit einzusetzen. Als solches kommen beispielsweise Nickelschwamm oder vorzugsweise feinteiliges Nickelpulver in Betracht. Derartiges großoberflächiges metallisches Nickel ist an sich bekannt und kommerziell erhältlich.

Erfindungsgemäß setzt man in Stufe a) weist 4 bis 10 mol, vorzugsweise 4 bis 8 mol, insbesondere 4 bis 6 mol, Verbindung (I) pro mol Ni(0) ein.

In einer vorteilhaften Ausführungsform kann man in Stufe a) als flüssiges Verdünnungsmittel eine Verbindung der Formel (I), ein olefinisch ungesättigtes Nitril, vorzugsweise ein Pentennitril, wie cis-2-Pentennitril, trans-2-Pentennitril, cis-3-Pentennitril, trans-3-Pentennitril, 4-Pentennitril, 2-Methyl-3-butennitril, Z-2-Methyl-2-butennitril, E-2-Methyl-2-butennitril, ein Dinitril, wie Adipodinitril, Methylglutaronitril, Aromaten, wie Benzol, Toluol, o-Xylol, m-Xylol, p-Xylol, Aliphaten, wie Cyclohexan, oder Gemische solcher Verbindungen einsetzen.

Vorteilhaft kann man die Herstellung des ersten Systems in Stufe a) in Gegenwart eines homogenen oder heterogenen, vorzugsweise homogenen Katalysator durchführen.

Als homogener Katalysator kommt vorteilhaft eine Protonsäure oder ein Gemisch solcher Protonsäuren, wie Chlorwasserstoffsäure, in Betracht.

Als homogener Katalysator kommt vorteilhaft eine Verbindung der Formel

(R¹X¹) (R²X²)PCl

oder

(R¹X¹)PCl₂

mit den für R¹, R², X¹, X² bereits oben genannten Bedeutungen
oder ein Gemisch solcher Verbindungen in Betracht.

Den in Stufe a) eingesetzten Katalysator kann man von Stufe a) in Stufe b) übertragen. Als vorteilhaft hat sich die Entfernung des Katalysators aus Stufe a) zwischen den Stufen a) und b).

In einer vorteilhaften Ausführungsform kann man 1 bis 4 mol, vorzugsweise 1 bis 3 mol Verbindung (II) pro mol Ni(0) einsetzen, wobei insbesondere im Falle von schwierig herstellbaren oder teuren Verbindungen (II) in einer besonderen Ausführungsform ein Verhältnis im Bereich von 1 bis 2 mol Verbindung (II) zu Ni(0) in Betracht kommen kann.

In einer vorteilhaften Ausführungsform kann man in Stufe b) als flüssiges Verdünnungsmittel eine Verbindung der Formel (I), ein olefinisch ungesättigtes Nitril, vorzugsweise ein Pentennitril, wie cis-2-Pentennitril, trans-2-Pentennitril, cis-3-Pentennitril, trans-3-Pentennitril, 4-Pentennitril, 2-Methyl-3-butennitril, Z-2-Methyl-2-butennitril, E-2-Methyl-2-butennitril, ein Dinitril, wie Adipodinitril, Methylglutaronitril, Aromaten, wie Benzol, Toluol, o-Xylol, m-Xylol, p-Xylol, Aliphaten, wie Cyclohexan, oder Gemische solcher Verbindungen einsetzen.

In einer besonders bevorzugten Ausführungsform kommt der Einsatz des gleichen flüssigen Verdünnungsmittel in Stufe a) und b) in Betracht.

Ein weiterer Gegenstand der Erfindung ist ein Verfahren zur Herstellung von Gemischen monoolefinischer C₅-Mononitrile mit nichtkonjugierter C=C- und C=N-Bindung durch Hydrocyanierung eines 1,3-Butadien-haltigen Kohlenwasserstoffgemisches in Gegenwart eines Katalysators, das dadurch gekennzeichnet ist, daß die Hydrocyanierung in Gegenwart mindestens eines der zuvor beschriebenen erfindungsgemäßen Systeme erfolgt.

Vorzugsweise wird zur Herstellung von monoolefinischen C₅-Mononitrilen nach dem erfindungsgemäßen Verfahren ein Kohlenwasserstoffgemisch eingesetzt, das einen 1,3-Butadien-Gehalt von mindestens 10 Vol.-%, bevorzugt mindestens 25 Vol.-%, insbesondere mindestens 40 Vol.-%, aufweist.

Zur Herstellung von Gemischen monoolefinischer C₅-Mononitrile, die z.B. 3-Pentennitril und 2-Methyl-3-butennitril enthalten und die als Zwischenprodukte für die Weiterverarbeitung zur Adipodinitril geeignet sind, kann man reines Butadien oder 1,3-Butadien-haltige Kohlenwasserstoffgemische einsetzen.

1,3-Butadien-haltige Kohlenwasserstoffgemische sind in großtechnischem Maßstab erhältlich. So fällt z.B. bei der Aufarbeitung von Erdöl durch Steamcracken von Naphtha ein als C₄-Schnitt bezeichnetes Kohlenwasserstoffgemisch mit einem hohen Gesamtolefinanteil an, wobei etwa 40 % auf 1,3-Butadien und der Rest auf Monoolefine und mehrfach ungesättigte Kohlenwasserstoffe sowie Alkane entfällt. Diese Ströme enthalten immer auch geringe Anteile von im allgemeinen bis zu 5 % an Alkinen, 1,2-Dienen und Vinylacetylen.

Reines 1,3-Butadien kann z. B. durch extraktive Destillation aus technisch erhältlichen Kohlenwasserstoffgemischen isoliert werden.

C₄-Schnitte werden gegebenenfalls von Alkinen, wie z. B. Propin oder Butin, von 1,2-Dienen, wie z. B. Propadien, und von Alkeninen, wie z. B. Vinylacetylen, im wesentlichen befreit. Ansonsten werden unter Umständen Produkte erhalten, bei denen eine C=C-Doppelbindung in Konjugation mit der C=N-Bindung steht. Aus "Applied Homogeneous Catalysis with Organometalic Compounds", Bd. 1, VCH Weinheim, S. 479 ist bekannt, dass das bei der Isomerisierung von 2-Methyl-3-butennitril und 3-Pentennitril entstehende, konjugierte 2-Pentennitril als ein Reaktionsinhibitor für die Zweitaddition von Cyanwasserstoff zu Adipinsäuredinitril wirkt. Es wurde festgestellt, daß die oben genannten, bei der Hydrocyanierung eines nicht vorbehandelten C₄-Schnittes erhaltenen konjugierten Nitrile auch als Katalysatorgifte für den ersten Reaktionsschritt der Adipinsäureherstellung, die Monoaddition von Cyanwasserstoff, wirken.

Daher entfernt man gegebenenfalls aus dem Kohlenwasserstoffgemisch solche Komponenten teilweise oder vollständig, die bei katalytischer Hydrocyanierung Katalysatorgifte ergeben, insbesondere Alkine, 1,2-Diene und Gemische davon. Zur Entfernung dieser Komponenten wird der C₄-Schnitt vor der Addition von Cyanwasserstoff einer katalytischen Teilhydrierung unterzogen. Diese Teilhydrierung erfolgt in Gegenwart eines Hydrierungskatalysators, der befähigt ist, Alkine und 1,2-Diene selektiv neben anderen Dienen und Monoolefinen zu hydrieren.

Geeignete heterogene Katalysatorsysteme umfassen im Allgemeinen eine Übergangsmetallverbindung auf einem inerten Träger. Geeignete anorganische Träger sind die hierfür üblichen Oxide, insbesondere Silicium- und Aluminiumoxide, Alumosilikate, Zeolithe, Carbide, Nitride etc. und deren Mischungen. Bevorzugt werden als Träger Al₂O₃, SiO₂ und deren Mischungen verwendet. Insbesondere handelt es sich bei den verwendeten heterogenen Katalysatoren um die in den US-A-4,587,369; US-A-4,704,492 und US-A-4,493,906 beschriebenen. Weiterhin geeignete Katalysatorsysteme auf Cu-Basis werden von der Fa. Dow Chemical als KLP-Katalysator vertrieben.

Die Addition von Cyanwasserstoff an 1,3-Butadien oder ein 1,3-Butadien-haltiges Kohlenwasserstoffgemisch, z. B. einen vorbehandelten, teilhydrierten C₄-Schnitt, kann kontinuierliche, semikontinuierlich oder diskontinuierlich erfolgen.

Nach einer geeigneten Variante des erfindungsgemäßen Verfahrens erfolgt die Addition des Cyanwasserstoffs kontinuierlich. Geeignete Reaktoren für die kontinuierliche Umsetzung sind dem Fachmann bekannt und werden z. B. in Ullmanns Enzyklopädie der technischen Chemie, Bd. 1, 3. Aufl., 1951, S. 743 ff. beschrieben. Vorzugsweise wird für die kontinuierliche Variante des erfindungsgemäßen Verfahrens eine Rührkesselkaskade oder ein Rohrreaktor verwendet.

Gemäß einer bevorzugten Variante des erfindungsgemäßen Verfahrens erfolgt die Addition des Cyanwasserstoffs an 1,3-Butadien oder ein 1,3-Butadien-haltiges Kohlenwasserstoffgemisch semikontinuierlich.

Das semikontinuierliche Verfahren umfasst:
a) Befüllen eines Reaktors mit dem Kohlenwasserstoffgemisch, gegebenenfalls einem Teil des Cyanwasserstoffs und einem gegebenenfalls in situ erzeugten, erfindungsgemäßen Hydrocyanierungskatalysator sowie gegebenenfalls einem Lösungsmittel,
b) Umsetzung des Gemisches bei erhöhter Temperatur und erhöhtem Druck, wobei bei semikontinuierlicher Fahrweise Cyanwasserstoff nach Maßgabe seines Verbrauchs eingespeist wird,
c) Vervollständigung des Umsatzes durch Nachreagieren und anschließende Aufarbeitung.

Geeignete druckfeste Reaktoren sind dem Fachmann bekannt und werden z. B. in Ullmanns Enzyklopädie der technischen Chemie, Bd. 1, 3. Auflage, 1951, S. 769 ff. beschrieben. Im allgemeinen wird für das erfindungsgemäße Verfahren ein Autoklav verwendet, der gewünschtenfalls mit einer Rührvorrichtung und einer Innenauskleidung versehen sein kann. Für die obigen Schritte gilt vorzugsweise folgendes zu beachten:
Schritt a):
   Der druckfeste Reaktor wird vor Beginn der Reaktion mit dem teilhydrierten C₄-Schnitt, Cyanwasserstoff einem Hydrocyanierungskatalysator sowie ggf. einem Lösungsmittel befüllt. Geeignete Lösungsmittel sind dabei die zuvor bei der Herstellung der erfindungsgemäßen Katalysatoren genannten, bevorzugt aromatischen Kohlenwasserstoffe, wie Toluol und Xylol, oder Tetrahydrofuran.
Schritt b):
   Die Umsetzung des Gemisches erfolgt im allgemeinen bei erhöhter Temperatur und erhöhtem Druck. Dabei liegt die Reaktionstemperatur im allgemeinen in einem Bereich von etwa 0 bis 200°C, bevorzugt etwa 50 bis 150°C. Der Druck liegt im allgemeinen in einem Bereich von etwa 1 bis 200 bar, bevorzugt etwa 1 bis 100 bar, insbesondere 1 bis 50 bar, insbesondere bevorzugt 1 bis 20 bar. Dabei wird während der Reaktion Cyanwasserstoff nach Maßgabe seines Verbrauchs eingespeist, wobei der Druck im Autoklaven im wesentlichen konstant bleibt. Die Reaktionszeit beträgt etwa 30 Minuten bis 5 Stunden.
Schritt c):
   Zur Vervollständigung des Umsatzes kann sich an die Reaktionszeit eine Nachreaktionszeit von 0 Minuten bis etwa 5 Stunden, bevorzugt etwa 1 Stunde bis 3,5 Stunden anschließen, in der kein Cyanwasserstoff mehr in den Autoklaven eingespeist wird. Die Temperatur wird in dieser Zeit im wesentlichen konstant auf der zuvor eingestellten Reaktionstemperatur belassen. Die Aufarbeitung erfolgt nach gängigen Verfahren und umfaßt die Abtrennung des nicht umgesetzten 1,3-Butadiens und des nicht umgesetzten Cyanwasserstoffs, z. B. durch Waschen oder Extrahieren und die destillative Aufarbeitung des übrigen Reaktionsgemisches zur Abtrennung der Wertprodukte und Rückgewinnung des noch aktiven Katalysators.

Gemäß einer weiteren geeigneten Variante des erfindungsgemäßen Verfahrens erfolgt die Addition des Cyanwasserstoffs an das 1,3-Butadien-haltige Kohlenwasserstoffgemisch diskontinuierlich. Dabei werden im wesentlichen die bei semikontinuierlichen Verfahren beschriebenen Reaktionsbedingungen eingehalten, wobei in Schritt b) kein zusätzlicher Cyanwasserstoff eingespeist, sondern dieser komplett vorgelegt wird.

Allgemein läßt sich die Herstellung von Adipinsäuredinitril aus einem Butadien-haltigen Gemisch durch Addition von 2 Moläquivalenten Cyanwasserstoff in drei Schritte gliedern:
1. Herstellung von C₅-Monoolefingemischen mit Nitrilfunktion.
2. Isomerisierung des in diesen Gemischen enthaltenen 2-Methyl-3-butennitrils zu 3-Pentennitril und Isomerisierung des so gebildeten und des in den Gemischen bereits aus Schritt 1 enthaltenen 3-Pentennitrils zu verschiedenen n-Pentennitrilen. Dabei soll ein möglichst hoher Anteil an 3-Pentennitril bzw. 4-Pentennitril und ein möglichst geringer Anteil an konjugiertem und gegebenenfalls als Katalysatorgift wirksamen 2-Pentennitril und 2-Methyl-2-butennitril gebildet werden.
3. Herstellung von Adipinsäuredinitril durch Addition von Cyanwasserstoff an das in Schritt 2 gebildete 3-Pentennitril welches zuvor "in situ" zu 4-Pentennitril isomerisiert wird. Als Nebenprodukte treten dabei z. B. 2-Methyl-glutarodinitril aus der Markownikow-Addition von Cyanwasserstoff an 4-Pentennitril oder der anti-Markownikow-Addition von Cyanwasserstoff an 3-Pentennitril und Ethylsuccinodinitril aus der Markownikow-Addition von Cyanwasserstoff an 3-Pentennitril auf.

Vorteilhafterweise eignen sich die erfindungsgemäßen Katalysatoren auf Basis von Phosphonitliganden auch für die Stellungs- und Doppelbindungsisomerisierung in Schritt 2 und/oder die Zweitaddition von Cyanwasserstoff in Schritt 3.

Nach einer geeigneten Ausführungsform des erfindungsgemäßen Verfahrens beträgt das bei der Monoaddition von Cyanwasserstoff an das 1,3-Butadien-haltige Kohlenwasserstoffgemisch erhaltene Mengenverhältnis von 3-Pentennitril zu 2-Methyl-3-butennitril mindestens 1,9:1, bevorzugt mindestens 2,1:1.

Vorteilhafterweise zeigen die erfindungsgemäß eingesetzten Katalysatoren nicht nur eine hohe Selektivität im Bezug auf die bei der Hydrocyanierung von 1,3-Butadien-haltigen Kohlenwasserstoffgemischen erhaltenen Monoadditionsprodukte, sondern sie können bei der Hydrocyanierung auch mit einem Überschuss an Cyanwasserstoff versetzt werden, ohne dass es zu einer merklichen Abscheidung von inaktiven Nickel(II)-Verbindungen, wie z.B. Nickel(II)-Cyanid, kommt. Im Gegensatz zu bekannten Hydrocyanierungskatalysatoren auf Basis nicht-komplexer Phosphin- und Phosphitliganden eignen sich die Katalysatoren der Formel I somit nicht nur für kontinuierliche Hydrocyanierungsverfahren, bei denen ein Cyanwasserstoffüberschuss im Reaktionsgemisch im allgemeinen wirkungsvoll vermieden werden kann, sondern auch für semikontinuierliche Verfahren und Batch-Verfahren, bei denen im allgemeinen ein starker Cyanwasserstoffüberschuss vorliegt. Somit weisen die erfindungsgemäß eingesetzten Katalysatoren und die auf ihnen basierenden Verfahren zur Hydrocyanierung im allgemeinen höhere Katalysatorrückführungsraten und längere Katalysatorstandzeiten auf als bekannte Verfahren. Dies ist neben einer besseren Wirtschaftlichkeit auch unter ökologischen Aspekten vorteilhaft, da das aus dem aktiven Katalysator mit Cyanwasserstoff gebildete Nickelcyanid stark giftig ist und unter hohen Kosten aufgearbeitet oder entsorgt werden muss.

Neben der Hydrocyanierung von 1,3-Butadien-haltigen Kohlenwasserstoffgemischen eignen sich die erfindungsgemäßen Systeme im allgemeinen für alle gängigen Hydrocyanierungsverfahren. Dabei sei insbesondere die Hydrocyanierung von nichtaktivierten Olefinen, z.B. von Styrol und 3-Pentennitril, genannt.

Eine weitere vorteilhafte Ausführungsform der Hydrocyanierung und Isomerisierung kann US 6,981,772 entnommen werden, mit der Maßgabe, das anstelle der in dieser Patentschrift genannten Katalysatoren ein erfindungsgemäßes System oder ein Gemisch solcher Systeme eingesetzt wird.

Eine weitere vorteilhafte Ausführungsform der Hydrocyanierung und Isomerisierung kann US 6,127,567 entnommen werden, mit der Maßgabe, das anstelle der in dieser Patentschrift genannten Katalysatoren ein erfindungsgemäßes System oder ein Gemisch solcher Systeme eingesetzt wird. Eine weitere vorteilhafte Ausführungsform der Hydrocyanierung kann US 5,693,843 entnommen werden, mit der Maßgabe, das anstelle der in dieser Patentschrift genannten Katalysatoren ein erfindungsgemäßes System oder ein Gemisch solcher Systeme eingesetzt wird.

Eine weitere vorteilhafte Ausführungsform der Hydrocyanierung kann US 5,523,453 entnommen werden, mit der Maßgabe, das anstelle der in dieser Patentschrift genannten Katalysatoren ein erfindungsgemäßes System oder ein Gemisch solcher Systeme eingesetzt wird.

Die Erfindung wird anhand der folgenden, nicht einschränkenden Beispiele näher erläutert.

### Beispiele

Die Ausbeuten wurden gaschromatographisch bestimmt (Säule: 30 m Stabil-Wachs, Temperaturprogramm: 5 Minuten isotherm bei 50°C, danach Aufheizen mit einer Geschwindigkeit von 5 °C/min auf 240°C, Gaschromatographie: Hewlett Packard HP 5890)

Sämtliche Beispiele wurden unter einer Schutzatmosphäre aus Argon durchgeführt. Die Abkürzung Nickel(0)-(m/p-Tolylphosphit) steht für eine Mischung enthaltend 2,35 Gew.-% Ni(0), 19 Gew.-% 3-Pentennitril und 78,65 Gew.-% m/p-Tolylphosphit mit einem m:p-Verhältnis von 2:1.

Als Chelatligand wurde eingesetzt:

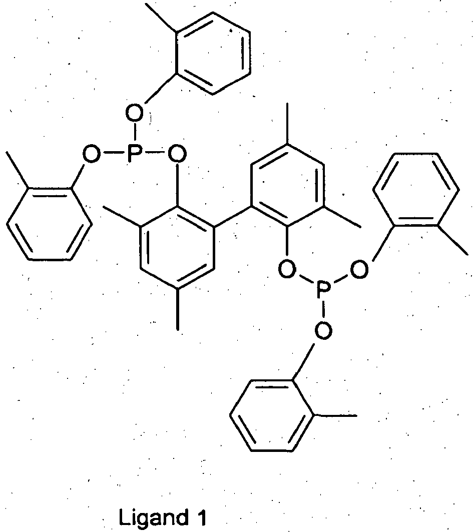

Ni(COD)₂ steht für Ni(0)-bis-(1,4-cyclooctadien).

In den Tabellen steht 2M3BN für 2-Methyl-3-butennitril, t2M2BN für trans-2-Methyl-2-butennitril, c2M2BN für cis-2-Methyl-2-butennitril, t2PN für trans-2-Pentennitril, 4PN für 4-Pentennitril, t3PN für trans-3-Pentennitril, c3PN für cis-3-Pentennitril, MGN für Methylglutaronitril und ADN für Adipodinitril.

### Beispiele 1-18: Einsatz von Ligand 1 als Verbindung (II)

### Beispiele 1-3: Isomerisierung von 2-Methyl-3-butennitril zu 3-Pentennitril

### Beispiel 1 (Vergleich)

1 mol-Äquivalent Ni(0)-(m/p-Tolylphosphit) (0,5 mmol Ni(0)) wurde mit 465 mol- Äquivalenten 2-Methyl-3-butennitril versetzt und auf 115°C erwärmt. Nach 90 Minuten und nach 180 Minuten wurden dem Reaktionsgemisch Proben entnommen und folgende Ausbeuten (in Prozent) bestimmt:

| Zeit | 2M3BN | t2M2BN | c2M2BN | t2PN | 4PN | t3PN | c3PN | 3PN/2M 3BN |
|---|---|---|---|---|---|---|---|---|
| 90 Min | 84,5 | 1,3 | 0,3 | | | 13,0 | | 0,15 |
| 180 Min | 72,4 | 1,5 | 0,5 | | | 24,4 | | 0,34 |

### Beispiel 2 (Vergleich)

1 mol-Äquivalent Ni(COD)₂ (0,58 mmol Ni(0)) wurde mit 3 mol-Äquivalent Ligand 1 und 465 mol-Äquivalent 2-Methyl-3-butennitril versetzt, 1 Stunde bei 25°C gerührt und dann auf 115°C erwärmt. Nach 90 Minuten und nach 180 Minuten wurden dem Reaktionsgemisch Proben entnommen und folgende Ausbeuten (in Prozent) bestimmt:

| Zeit | 2M3BN | t2M2B N | c2M2B N | t2PN | 4PN | t3PN | c3PN | 3PN/2 M3BN |
|---|---|---|---|---|---|---|---|---|
| 90 Min | 4,69 | 1,4 | 0,22 | 0,3 | 0,78 | 87,82 | 4,80 | 19,74 |
| 180 Min | 4,52 | 1,34 | 0,16 | 0,23 | 1,41 | 85,3 | 7,0 | 20,42 |

### Beispiel 3 (erfindungsgemäß)

1 mol-Äquivalent Ni (0)- (m/p-Tolylphosphit) (0,4 mmol Ni(0)) wurde mit 1 mol-Äquivalent Ligand 1 und 465 mol-Äquivalent 2-Methyl-3-butennitril versetzt, 12 Stunde bei 25°C gerührt und dann auf 115°C erwärmt. Nach 90 Minuten und nach 180 Minuten wurden dem Reaktionsgemisch Proben entnommen und folgende Ausbeuten (in Prozent) bestimmt:

| Zeit | 2M3BN | T2M2B N | c2M2B N | t2PN | 4PN | T3PN | c3PN | 3PN/2 M3BN |
|---|---|---|---|---|---|---|---|---|
| 90 Min | 28,81 | 1,5 | | | 0,1 | 57,6 | | 2 |
| 180 Min | 13,31 | 1,3 | | | 0,1 | 75,5 | | 5,68 |

### Beispiele 4-15: Hydrocyanierung von 3-Pentennitril zu Adiponitril Beispiel 4 (Vergleich)

1 mol-Äquivalent Nickel(0)-(m/p-Tolylphosphit) (0,6 mmol Ni(0)) wurde mit 365 mol-Äquivalenten 3-Pentennitril versetzt, eine Stunde bei 25°C gerührt und auf 70°C erwärmt. Zu dieser Mischung wurde 1 mol-Äquivalent ZnCl₂ gegeben und weitere 5 Minuten gerührt. In einem Argon-Trägergasstrom wurden 94 mol-Äquivalente HCN/Stunde*Ni eingegast. Nach 30 Minuten, 60 Minuten und 150 Minuten wurden Proben entnommen und folgende Ausbeuten (in Prozent) bestimmt:

| Zeit | MGN | ADN | ADN-Selektivität (%) |
|---|---|---|---|
| 30 Min | 3,35 | 10,75 | 76,2 |
| 60 Min | 6,87 | 26,39 | 79,3 |
| 150 Min | 7,11 | 27,82 | 79,6 |

### Beispiel 5 (Vergleich)

Es wurde verfahren wie in Beispiel 4 mit der Ausnahme, daß anstelle von 1 mol-Äquivalent Nickel(0)-(m/p-Tolylphosphit) 1 mol-Äquivalent Ni(COD)₂ (0,27 mmol Ni(0)) und 1 mol-Äquivalent Ligand 1 eingesetzt wurde.

Nach 30 Minuten, 60 Minuten und 150 Minuten wurden Proben entnommen und folgende Ausbeuten (in Prozent) bestimmt:

| Zeit | MGN | ADN | ADN-Selektivität (%) |
|---|---|---|---|
| 30 Min | 0,68 | 2,19 | 76,2 |
| 60 Min | 0,99 | 6,17 | 86,2 |
| 120 Min | 1.01 | 7,28 | 87,8 |

### Beispiel 6 (Vergleich)

Es wurde verfahren wie in Beispiel 5 (mit 0,64 mmol Ni(0)) mit der Ausnahme, daß anstelle von 94 mol-Äquivalenten HCN/Stunde*Ni nur 38 mol-Äquivalenten HCN/Stunde*Ni eingegast wurden.

Nach 30 Minuten, 60 Minuten und 150 Minuten wurden Proben entnommen und folgende Ausbeuten (in Prozent) bestimmt:

| Zeit | MGN | ADN | ADN-Selektivität (%) |
|---|---|---|---|
| 30 Min | 0,88 | 1,33 | 60,0 |
| 60 Min | 1,71 | 8,69 | 83,5 |
| 120 Min | 2,01 | 15,90 | 88,7 |

### Beispiel 7 (erfindungsgemäß)

1 mol-Äquivalent Ni(COD)₂ (0,61 mmol Ni(0)) wurde mit 1 mol-Äquivalent Ligand 1, 4 mol-Äquivalenten m/p-Tolylphosphit (m:p = 2:1) und 365 mol-Äquivalenten 3-Pentennitril versetzt, eine Stunde bei 25°C gerührt und auf 70°C erwärmt. Zu dieser Mischung wurde 1 mol-Äquivalent ZnCl₂ zugegeben und weitere 5 Minuten gerührt. In einem Argon-Trägergasstrom wurden 133 mol-Äquivalente HCN/Stunde*Ni eingegast.

Nach 30 Minuten, 60 Minuten und 150 Minuten wurden Proben entnommen und folgende Ausbeuten (in Prozent) bestimmt:

| Zeit | MGN | ADN | ADN-Selektivität (%) |
|---|---|---|---|
| 30 Min | 2,86 | 17,50 | 85,9 |
| 60 Min | 3,96 | 36,86 | 90,3 |
| 120 Min | 6,88 | 77,27 | 91,8 |

### Beispiel 8 (erfindungsgemäß)

Es wurde wie in Beispiel 7 verfahren (mit 0,53 mmol Ni(0)) mit der Ausnahme, daß anstelle von 133 mol-Äquivalente HCN/Stunde*Ni 28 mol-Äquivalente HCN/Stunde*Ni eingegast wurden.

Nach 30 Minuten, 60 Minuten und 150 Minuten wurden Proben entnommen und folgende Ausbeuten (in Prozent) bestimmt:

| Zeit | MGN | ADN | ADN-Selektivität (%) |
|---|---|---|---|
| 30 Min | 0,49 | 8,02 | 94,2 |
| 60 Min | 1,10 | 19,73 | 94,7 |
| 120 Min | 1,88 | 33,54 | 94,7 |

### Beispiel 9 (erfindungsgemäß)

1 mol-Äquivalent Nickel(0)-(m-/p-Tolylphosphit) (0,6 mmol Ni(0)) wurde mit 1,2 mol-Äquivalent Ligand 1 und 365 mol-Äquivalent 3-Pentennitril versetzt, 12 Stunden bei 25°C gerührt und auf 70°C erwärmt. Zu dieser Mischung wurde 1 mol-Äquivalent ZnCl₂ zugegeben und weitere 5 Min. gerührt. In einem Ar-Trägergasstrom wurden dann 131 mol-Äquivalent HCN/h*Ni eingegast.

Nach 30 Min., 60 Min. und 120 Min. wurden Proben entnommen und folgende Ausbeuten (in Prozent) bestimmt:

| Zeit | MGN | ADN | ADN-Selektivität (%) |
|---|---|---|---|
| 30 Min | 1,67 | 15,21 | 90,1 |
| 60 Min | 3,13 | 39,05 | 92,6 |
| 120 Min | 5,15 | 65,04 | 92,7 |

### Beispiel 10 (Vergleich)

1 mol-Äquivalent Ni(COD)₂ (0,49 mmol Ni(0)) wurde mit 3 mol-Äquivalent Ligand 1 und 365 mol-Äquivalent 3-Pentennitril versetzt, eine Stunde bei 25°C gerührt und auf 70°C erwärmt. Zu dieser Mischung wurde 1 mol-Äquivalent ZnCl₂ zugegeben und weitere 5 Min. gerührt. In einem Ar-Trägergasstrom wurden dann 43 mol-Äquivalente HCN/h*Ni eingegast.

Nach 60 Min. und 120 Min. wurden Proben entnommen und folgende Ausbeuten (in Prozent) bestimmt:

| Zeit | MGN | ADN | ADN-Selektivität (%) |
|---|---|---|---|
| 60 Min | 2,41 | 11,73 | 83,0 |
| 120 Min | 3,21 | 29,14 | 90,1 |

### Beispiel 11 (Vergleich)

Es wurde wie in Beispiel 10 verfahren (mit 0,58 mmol Ni(0)) mit der Ausnahme, daß anstelle von 43 mol-Äquivalente HCN/h*Ni 95 mol-Äquivalente HCN/h*Ni eingegast wurden.

Nach 30 Min., 60 Min. und 120 Min. wurden Proben entnommen und folgende Ausbeuten (in Prozent) bestimmt:

| Zeit | MGN | ADN | ADN-Selektivität (%) |
|---|---|---|---|
| 30 Min | 1,40 | 13,32 | 90,5 |
| 60 Min | 2,26 | 31,96 | 93,4 |
| 120 Min | 3,69 | 58,46 | 94,0 |

### Beispiel 12 (Vergleich)

Es wurde wie in Beispiel 10 verfahren (mit 0,58 mmol Ni(0)) mit der Ausnahme, daß das Katalysatorgemisch anstelle von 1 Stunde 12 Stunden bei 25°C gerührt wurde und anstelle von 43 mol-Äquivalente HCN/h*Ni 122 mol-Äquivalente HCN/h*Ni eingegast wurden.

Nach 30 Min., 60 Min. und 180 Min. wurden Proben entnommen und folgende Ausbeuten (in Prozent) bestimmt:

| Zeit | MGN | ADN | ADN-Selektivität (%) |
|---|---|---|---|
| 30 Min | 1,71 | 18,50 | 91,5 |
| 60 Min | 2,52 | 36,10 | 93,5 |
| 180 Min | 5,92 | 91,04 | 93,9 |

### Beispiel 13 (Vergleich)

Es wurde wie in Beispiel 12 verfahren (mit 0,4 mmol Ni(0)) mit der Ausnahme, daß anstelle von 43 mol-Äquivalente HCN/h*Ni 150 mol-Äquivalente HCN/h*Ni eingegast wurden.

Nach 30 Min., 60 Min. und 120 Min. wurden Proben entnommen und folgende Ausbeuten (in Prozent) bestimmt:

| Zeit | MGN | ADN | ADN-Selektivität (%) |
|---|---|---|---|
| 30 Min | 3,47 | 42,03 | 92,4 |
| 60 Min | 4,90 | 67,36 | 93,2 |
| 120 Min | 5,96 | 83,92 | 93,4 |

### Beispiel 14 (erfindungsgemäß)

1 mol-Äquivalent Nickel(0)-(m-/p-Tolylphosphit) (0,6 mmol Ni(0)) wurde mit 3 mol-Äquivalenten Ligand 1 und 365 mol-Äquivalenten 3-Pentennitril versetzt, 12 Stunden bei 25°C gerührt und auf 70°C erwärmt. Zu dieser Mischung wurde 1 mol-Äquivalent ZnCl₂ zugegeben und weitere 5 Min. gerührt. In einem Ar-Trägergasstrom wurden dann 111 mol-Äquivalente HCN/h*Ni eingegast.

Nach 30 Min., 60 Min. und 120 Min. wurden Proben entnommen und folgende Ausbeuten (in Prozent) bestimmt:

| Zeit | MGN | ADN | ADN-Selektivität (%) |
|---|---|---|---|
| 30 Min | 1,11 | 16,31 | 93,6 |
| 60 Min | 2,11 | 36,31 | 94,5 |
| 120 Min | 4,14 | 70,50 | 94,5 |

### Beispiel 15 (erfindungsgemäß)

Es wurde verfahren wie in Beispiel 14 (mit 0,6 mmol Ni(0)) mit der Ausnahme, daß anstelle von 111 mol-Äquivalente HCN/h*Ni 109 mol-Äquivalente HCN/h*Ni eingegast wurden.

Nach 30 Min., 60 Min. und 120 Min. wurden Proben entnommen und folgende Ausbeuten (in Prozent) bestimmt:

| Zeit | MGN | ADN | ADN-Selektivität (%) |
|---|---|---|---|
| 30 Min | 1,03 | 15,79 | 93,9 |
| 60 Min | 2,00 | 34,31 | 94,5 |
| 120 Min | 4,58 | 77,68 | 94,4 |

### Beispiele 16-18: Hydrocyanierung von 1,3-Butadien zu 3-Pentennitril

### Beispiel 16 (Vergleich)

1 mol-Äquivalent Nickel(0)-(m-/p-Tolylphosphit) (1 mmol Ni(0)) wurde mit 500 mol-Äquivalent 1,3-Butadien und 420 mol-Äquivalenten HCN in THF versetzt, bei 25°C in einen Glasautoklaven gefüllt und auf 80°C erwärmt.

Mit einem Innenthermometer wurde folgender Druck- und Temperaturverlauf der Reaktion (leicht exotherme Reaktion) bestimmt:

| Zeit | Innentemperatur |
|---|---|
| 30 Min | 80,3 |
| 50 Min | 80,5 |
| 60 Min | 80,4 |
| 180 Min | 80,3 |

Nach 180 Minuten betrug der HCN-Umsatz zu 2-Methyl-3-butennitril und 3-Pentennitril 9,8 %. Das Verhältnis 2-Methyl-3-butennitril zu 3-Pentennitril betrug 1/3,4.

### Beispiel 17 (Vergleich)

1 mol-Äquivalent Ni(COD)₂ (0,32 mmol Ni(0)) wurde mit 3 mol-Äquivalent Ligand 1 in THF 20 Min. gerührt. Diese Lösung wurde mit 696 mol-Äquivalent 1,3-Butadien und 580 mol-Äquivalent HCN in THF versetzt, bei 25°C in einen Glasautoklaven gefüllt und auf 80°C erwärmt.

Mit einem Innenthermometer wurde folgender Druck- und Temperaturverlauf der Reaktion (leicht exotherme Reaktion) bestimmt:

| Zeit | Innentemperatur |
|---|---|
| 30 Min | 81,9 |
| 45 Min | 82 |
| 60 Min | 81,9 |
| 90 Min | 81,3 |
| 180 Min | 80,8 |

Nach 180 Minuten betrug der HCN-Umsatz zu 2-Methyl-3-butennitril und 3-Pentennitril 94,4%. Das molare Verhältnis 2-Methyl-3-buten-nitril zu 3-Pentennitril betrug 1/1,3.

### Beispiel 18 (erfindungsgemäß)

1 mol-Äquivalent Nickel(0)-(m-/p-Tolylphosphit) (1 mmol Ni(0)) wurde mit 1,2 mol-Äquivalent Ligand 1 in THF 12 h gerührt. Diese Lösung wurde mit 480 mol-Äquivalent 1,3-Butadien und 400 mol-Äquivalent HCN in THF versetzt, bei 25°C in einen Glasautoklaven gefüllt und auf 80°C erwärmt.

Mit einem Innenthermometer wurde folgender Druck- und Temperaturverlauf der Reaktion (leicht exotherme Reaktion) bestimmt:

| Zeit | Innentemperatur |
|---|---|
| 30 Min | 86 |
| 45 Min | 88,6 |
| 60 Min | 86,9 |
| 120 Min | 80 |

Nach 180 Minuten betrug der HCN-Umsatz zu 2-Methyl-3-butennitril und 3-Pentennitril mehr als 99 %. Das molare Verhältnis 2-Methyl-3-butennitril zu 3-Pentennitril betrug 1/1,5.

### Beispiele 19-25: Einsatz von Ligand 2 als Verbindung (II)

### Beispiele 19-20: Isomerisierung von 2-Methyl-3-butennitril zu 3-Pentennitril

### Beispiel 19 (Vergleich)

1 mol-Äquivalente Ni (COD) ₂ (0,58 mmol Ni(0)) wurden mit 3 mol-Äquivalent Ligand 2 und 465 mol-Äquivalent 2-Methyl-3-butennitril versetzt, 1h bei 25°C gerührt und auf 115°C erwärmt.

Nach 90 Minuten und 180 Minuten wurden Proben entnommen und folgende Ausbeuten (in Prozent) bestimmt:

| Zeit | 2M3BN | t2M2B N | c2M2 | t2PBN N | 4PN | t3PN | c3PN | 3PN/2 M3BN |
|---|---|---|---|---|---|---|---|---|
| 90 Min | 11,96 | 1,81 | 0,30 | 0,27 | | 82,75 | 2,48 | |
| 180 Min | 4,77 | 1,81 | 0,33 | 0,18 | 1,32 | 86,6 | 4,88 | |

### Beispiel 20 (nicht erfindungsgemäß)

1 mol-Äquivalent Nickel(0)-(m-/p-Tolylphosphit) (0,4 mmol Ni(0)) wurde mit 1 mol-Äquivalent Ligand 2 und 465 mol-Äquivalent 2-Methyl-3-butennitril versetzt, 12h bei 25°C gerührt und auf 115°C erwärmt.

Nach 90 Minuten und 180 Minuten wurden Proben entnommen und folgende Ausbeuten (in Prozent) bestimmt:

| Zeit | 2M3BN | t2M2B N | c2M2B N | t2PN | 4PN | t3PN | c3PN | 3PN/2 M3BN |
|---|---|---|---|---|---|---|---|---|
| 90 Min | 59,96 | 1,78 | | 0,32 | 0,1 | 26,45 | | 0,44 |
| 180 Min | 44,09 | 2,30 | | 0,36 | 0,1 | 40,84 | | 0,93 |

### Beispiele 21-23: Hydrocyanierung von 3-Pentennitril zu Adipodinitril

### Beispiel 21 (Vergleich)

1 mol-Äquivalent Ni(COD)₂ (0,55 mmol Ni(0)) wurde mit 3 mol-Äquivalent Ligand 2 und 365 mol-Äquivalent 3-Pentennitril versetzt, eine Stunde bei 25°C gerührt und auf 70°C erwärmt. Zu dieser Mischung wurde 1 mol-Äquivalent ZnCl₂ zugegeben und weitere 5 Min. gerührt. In einem Ar-Trägergasstrom wurden dann 142 mol-Äquivalente HCN/h*Ni eingegast.

Nach 30 Minuten und 60 Minuten wurden Proben entnommen und folgende Ausbeuten (in Prozent) bestimmt:

| Zeit | MGN | ADN | ADN-Selektivität (%) |
|---|---|---|---|
| 30 Min | 1,80 | 18,91 | 91,3 |
| 60 Min | 2,51 | 32,57 | 92,9 |

### Beispiel 22 (nicht erfindungsgemäß)

1 mol-Äquivalent Ni(COD)₂ (0,49 mmol Ni(0)) wurde mit 1,2 mol-Äquivalent Ligand 2, 4 mol-Äquivalent m-/p-Tolylphosphit (m/p = 2:1) und 365 mol-Äquivalent 3-Pentennitril versetzt, eine Stunde bei 25°C gerührt und auf 70°C erwärmt. Zu dieser Mischung wurde 1 mol-Äquivalent ZnCl₂ zugegeben und weitere 5 Min. gerührt. In einem Ar-Trägergasstrom wurden dann 125 mol-Äquivalent HCN/h*Ni eingegast.

Nach 45 Minuten und 60 Minuten wurden Proben entnommen und folgende Ausbeuten (in Prozent) bestimmt:

| Zeit | MGN | ADN | ADN-Selektivität (%) |
|---|---|---|---|
| 45 Min | 1,85 | 21,51 | 92,1 |
| 60 Min | 2,29 | 27,58 | 92,3 |

### Beispiel 23 (nicht erfindungsgemäß)

1 mol-Äquivalent Nickel(0)-(m-/p-Tolylphosphit) (0,6 mmol Ni(0)) wurde mit 1 mol-Äquivalent Ligand 2 und 365 mol-Äquivalent 3-Pentennitril versetzt, 12 Stunden bei 25°C gerührt und auf 70°C erwärmt. Zu dieser Mischung wurde 1 mol-Äquivalent ZnCl₂ zugegeben und weitere 5 Min. gerührt. In einem Ar-Trägergasstrom wurden dann 120 mol-Äquivalent HCN/h*Ni eingegast.

Nach 30 Minuten und 60 Minuten wurden Proben entnommen und folgende Ausbeuten (in Prozent) bestimmt:

| Zeit | MGN | ADN | ADN-Selektivität (%) |
|---|---|---|---|
| 30 Min | 1,22 | 11,49 | 90,4 |
| 60 Min | 2,88 | 26,12 | 90,0 |

### Beispiele 24-25: Hydrocyanierung von 1,3-Butadien zu 3-Pentennitril

### Beispiel 24 (Vergleich)

1 mol-Äquivalent Ni(COD)₂ (1 mmol Ni(0)) wird mit 3 mol-Äquivalent Ligand 2 in THF 20 Min. gerührt. Diese Lösung wurde mit 557 mol-Äquivalent 1,3-Butadien und 433 mol-Äquivalent HCN in THF versetzt, bei 25°C in einen Glasautoklaven gefüllt und auf 80°C erwärmt.

Mit einem Innenthermometer wurde folgender Druck- und Temperaturverlauf der Reaktion (leicht exotherme Reaktion) bestimmt:

| Zeit | Innentemperatur |
|---|---|
| 15 Min | 82,2 |
| 30 Min | 82,1 |
| 120 Min | 81,1 |

Nach 180 Minuten betrug der HCN-Umsatz zu 2-Methyl-3-butennitril und 3-Pentennitril 97,5%. Das molare Verhältnis 2-Methyl-3-butennitril zu 3-Pentennitril betrug 1,5/1.

### Beispiel 25 (nicht erfindungsgemäß)

1 mol-Äquivalent Nickel(0)-(m-/p-Tolylphosphit) (1 mmol Ni(0)) wurde mit 1,2 mol-Äquivalent Ligand 2 in THF 12 h gerührt. Diese Lösung wurde mit 480 mol-Äquivalent 1,3-Butadien und 400 mol-Äquivalent HCN in THF versetzt, bei 25°C in einen Glasautoklaven gefüllt und auf 80°C erwärmt.

Mit einem Innenthermometer wurde folgender Druck- und Temperaturverlauf der Reaktion (leicht exotherme Reaktion) bestimmt:

| Zeit | Innentemperatur |
|---|---|
| 30 Min | 83,6 |
| 60 Min | 84,6 |
| 120 Min | 84,4 |
| 180 Min | 80,5 |

Nach 180 Minuten betrug der HCN-Umsatz zu 2-Methyl-3-butennitril und 3-Pentennitril über 99 %. Das molare Verhältnis 2-Methyl-3-butennitril zu 3-Pentennitril betrug 1,35/1.

### Beispiele 26-28: Einsatz von Ligand 3 als Verbindung (II)

### Beispiel 26 (Vergleich)

1 mol-Äquivalent Ni(COD)₂ (1 mmol N(0)) wurde mit 1,2 mol-Äquivalent Ligand 3 in THF 20 Min. gerührt. Diese Lösung wurde mit 480 mol-Äquivalent 1,3-Butadien und 400 mol-Äquivalent HCN in THF versetzt, bei 25°C in einen Glasautoklaven gefüllt und auf 80°C erwärmt.

Mit einem Innenthermometer wurde folgender Druck- und Temperaturverlauf der Reaktion (leicht exotherme Reaktion) bestimmt:

| Zeit | Innentemperatur |
|---|---|
| 5 Min | 85 |
| 10 Min | 89 |
| 15 Min | 92,9 |
| 20 Min | 90,3 |
| 30 Min | 86,1 |
| 60 Min | 82 |
| 120 Min | 81 |

Nach 180 Minuten betrug der HCN-Umsatz zu 2-Methyl-3-butennitril und 3-Pentennitril 88,0 %. Das molare Verhältnis 2-Methyl-3-butennitril zu 3-Pentennitril betrug 3/1.

### Beispiel 27 (nicht erfindungsgemäß)

1 mol-Äquivalent Nickel(0)-(m-/p-Tolylphosphit) (1 mmol Ni(0)) wurde mit 1,2 mol-Äquivalent Ligand 3 in THF 12 h gerührt. Diese Lösung wurde mit 462 mol-Äquivalent 1,3-Butadien und 390 mol-Äquivalent HCN in THF versetzt, bei 25°C in einen Glasautoklaven gefüllt und auf 80°C erwärmt.

Mit einem Innenthermometer wurde folgender Druck- und Temperaturverlauf der Reaktion (leicht exotherme Reaktion) bestimmt:

| Zeit | Innentemperatur |
|---|---|
| 30 Min | 91 |
| 40 Min | 122 |
| 50 Min | 84 |
| 60 Min | 80,2 |
| 120 Min | 80,2 |

Nach 180 Minuten betrug der HCN-Umsatz zu 2-Methyl-3-butennitril und 3-Pentennitril über 99 %. Das molare Verhältnis 2-Methyl-3-butennitril zu 3-Pentennitril betrug 2,5/1.

### Beispiel 28 (nicht erfindungsgemäß)

Es wurde verfahren wie in Beispiel 28 (mit 1 mmol Ni(0)) mit der Ausnahme, daß anstelle von 462 mol-Äquivalent 1,3-Butadien und 390 mol-Äquivalent HCN 720 mol-Äquivalente 1,3-Butadien und 600 mol-Äquivalente HCN eingesetzt wurden.

Mit einem Innenthermometer wurde folgender Druck- und Temperaturverlauf der Reaktion (leicht exotherme Reaktion) bestimmt:

| Zeit | Innentemperatur |
|---|---|
| 25 Min | 84 |
| 45 Min | 89,1 |
| 65 Min | 90,5 |
| 80 Min | 80,5 |
| 120 Min | 80,2 |

Nach 180 Minuten betrug der HCN-Umsatz zu 2-Methyl-3-butennitril und 3-Pentennitril 96,6 %. Das molare Verhältnis 2-Methyl-3-butennitril zu 3-Pentennitril betrug 2,8/1.

## Patentansprüche

1. Als Katalysator geeignetes System enthaltend
a) Ni(0)
b) 4 bis 10 mol pro mol Ni(0) gemäß a) eine Verbindung (I) der Formel
p(X¹R¹) (X²R²) (X³R³) (I)
mit
X¹, X², X³ Sauerstoff
R¹, R², R³ unabhängig voneinander gleiche oder unterschiedliche organische Reste
und
c) 1 bis 4 mol pro mol Ni(0) gemäß a) eine Verbindung (II) der Formel 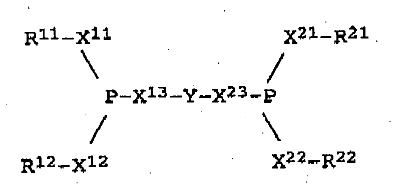 mit
X¹¹, X¹², X¹³ X²¹, X²², X²³ Sauerstoff
R¹¹, R¹² unabhängig voneinander gleiche oder unterschiedliche, einzelne oder verbrückte organische Reste
R²¹, R²² unabhängig voneinander gleiche oder unterschiedliche, einzelne oder verbrückte organische Reste
Y Brückengruppe
R¹, R², R³ unabhängig voneinander Phenyl-, o-Tolyl-, m-Tolyl-, oder p-Tolyl-Gruppe

2. System nach Anspruch 1, wobei R¹, R² und R³ unabhängig voneinander eine Phenyl-, o-Tolyl-, m-Tolyl- oder p-Tolyl-Gruppe darstellen, mit der Maßgabe, daß die Zahl der Phenyl-Gruppen in Verbindung (I) maximal 2 ist und die Zahl der o-Tolyl-Gruppen in Verbindung (I) maximal 2 ist.

3. System nach Anspruch 1, wobei man als Verbindung (I) eine solche der Formel
(o-Tolyl-O-)_{w} (m-Tolyl-O-)ₓ (p-Tolyl-O-)_{y} (Phenyl-O-)_{z} P mit w, x, y, z eine natürliche Zahl
mit w + x + y + z = 3 und
w, z kleiner gleich 2
einsetzt.

4. System nach den Ansprüchen 1 bis 3, wobei Y ein substituiertes oder unsubstituiertes Pyrocatechol, Bis(phenol) oder Bis(naphthol) ist.

5. System nach den Ansprüchen 1 bis 4, enthaltend einen Ni(0)-Komplex der Formel
Ni(0) (Verbindung(I))ₓ (Verbindung(II))
mit x = 1, 2.

6. Verfahren zur Herstellung eines Systems gemäß den Ansprüchen 1 bis 5, **dadurch gekennzeichnet, daß** man
a) Ni(0) mit einer Verbindung (I) in Gegenwart eines flüssigen Verdünnungsmittels umsetzt unter Erhalt eines ersten Systems enthaltend Ni(0) und Verbindung (I) und dann
b) dieses erste System mit einer Verbindung (II) in Gegenwart eines flüssigen Verdünnungsmittels umsetzt unter Erhalt eines Systems gemäß den Ansprüchen 1 bis 10.

7. Verfahren nach Anspruch 6, wobei man in Stufe a) als Ni(0) metallisches Nickel einsetzt.

8. Verfahren nach Anspruch 6 oder 7, wobei man in Stufe a) 4 bis 10 mol Verbindung (I) pro mol Ni(0) einsetzt.

9. Verfahren nach den Ansprüchen 6 bis 8, wobei man in Stufe a) als flüssiges Verdünnungsmittel eine Verbindung der Formel (I), 3-Pentennitril, 4-Pentennitril, 2-Methyl-3-Butennitril, Adipodinitril, Methylglutaronitril, Aromaten, Aliphaten oder Gemische hiervon einsetzt.

10. Verfahren nach den Ansprüchen 6 bis 9, wobei man die Herstellung des ersten Systems in Stufe a) in Gegenwart eines Katalysators durchführt.

11. Verfahren nach den Ansprüchen 6 bis 9, wobei man die Herstellung des ersten Systems in Stufe a) in Gegenwart eines homogenen Katalysators durchführt.

12. Verfahren nach den Ansprüchen 6 bis 9, wobei man die Herstellung des ersten Systems in Stufe a) in Gegenwart einer Protonsäure als homogenem Katalysator durchführt.

13. Verfahren nach den Ansprüchen 6 bis 9, wobei man die Herstellung des ersten Systems in Stufe a) in Gegenwart von Chlorwasserstoff als homogenem Katalysator durchführt.

14. Verfahren nach den Ansprüchen 6 bis 9, wobei man die Herstellung des ersten Systems in Stufe a) in Gegenwart einer Verbindung der Formel (R¹X¹) (R²X²)PCl oder (R¹X¹)PCl₂, wobei X¹, X², R¹, R² die Bedeutungen gemäß den Ansprüchen 1 bis 8 aufweisen, als homogenem Katalysator durchführt.

15. Verfahren nach den Ansprüchen 6 bis 14, wobei man zwischen Stufe a) und b) den Katalysator entfernt.

16. Verfahren nach den Ansprüchen 6 bis 15, wobei man in Stufe b) 1 bis 4 mol Vebindung (II) pro mol Ni(0) einsetzt.

17. Verfahren nach den Ansprüchen 6 bis 16, wobei man in Stufe b) als flüssiges Verdünnungsmittel eine Verbindung der Formel (I), 3-Pentennitril, 4-Pentennitril, 2-Methyl-3-Butennitril, Adipodinitril, Methylglutaronitril, Aromaten, Aliphaten oder Gemische hiervon einsetzt.

18. Verfahren nach den Ansprüchen 6 bis 17, wobei man in Stufe a) und Stufe b) das gleiche flüssige Verdünnungsmittel einsetzt.

19. Verfahren zur Herstellung von Gemischen monoolefinischer C₅-Mononitrile mit nichtkonjugierter C=C- und C=N-Bindung durch Hydrocyanierung eines 1,3-Butadien-haltigen Kohlenwasserstoffgemisches in Gegenwart eines Katalysators, **dadurch gekennzeichnet, daß** man die Hydrocyanierung in Gegenwart mindestens eines der Systeme gemäß den Ansprüchen 1 bis 5 durchführt.

20. Verfahren zur Herstellung eines Dinitrils durch Hydrocyanierung eines Gemischs monooelfinischer C₅-Mononitrile mit nichtkonjugierter C=C- und C=N-Bindung in Gegenwart eines Katalysators, **dadurch gekennzeichnet, daß** man die Hydrocyanierung in Gegenwart mindestens eines der Systeme gemäß den Ansprüchen 1 bis 5 durchführt.

21. Verfahren zur Herstellung von Adipodinitril durch Hydrocyanierung eines Gemischs monooelfinischer C₅-Mononitrile mit nichtkonjugierter C=C- und C=N-Bindung in Gegenwart eines Katalysators, **dadurch gekennzeichnet, daß** man die Hydrocyanierung in Gegenwart mindestens eines der Systeme gemäß den Ansprüchen 1 bis 5 durchführt.

## Claims

1. A system comprising
a) Ni(0)
b) from 4 to 10 mol per mol of Ni(0) in a) of a compound (I) of the formula
P(X¹R¹)(X²R²)(X³R³) (I)
where
X¹, X², X³ are each oxygen
R¹, R², R³ are, independently of one another, identical or different organic radicals
and
c) from 1 to 4 mol per mol of Ni (0) in a) of a compound (II) of the formula 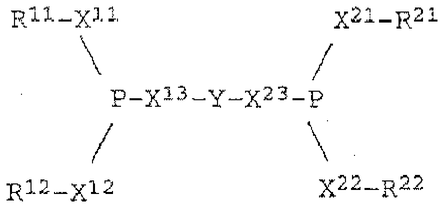 where
X¹¹, X¹², X¹³, X²¹, X²², X²³ are each oxygen,
R¹¹, R¹² are, independently of one another, identical or different, individual or bridged organic radicals,
R²¹, R²² are, independently of one another, identical or different, individual or bridged organic radicals and
Y is a bridging group and
R¹, R² and R³ are each, independently of one another, a phenyl, o-tolyl, m-tolyl or p-tolyl group,
which is suitable as catalyst.

2. The system according to claim 1, wherein R¹, R² and R³ are each, independently of one another, a phenyl, o-tolyl, m-tolyl or p-tolyl group, with the proviso that the number of phenyl groups in compound (I) is not more 2 and the number of otolyl groups in compound (I) is not more than 2.

3. The system according to claim 1, wherein the compound (I) used is a compound of the formula (o-tolyl-O-)_{w} (m-tolyl-O-)ₓ (p-tolyl-O-)_{y} (phenyl-O-)_{z} P
where w, x, y, z are each a natural number
and w + x + y + z = 3 and
w, z are each less than or equal to 2.

4. The system according to any of claims 1 to 3, wherein Y is a substituted or unsubstituted pyrocatechol, bis(phenol) or bis(naphthol).

5. The system according to any of claims 1 to 4, comprising an Ni(0) complex of the formula
Ni(0) (compound (I))ₓ(compound(II))
where x = 1, 2.

6. A process for preparing a system according to any of claims 1 to 5, which comprises
a) reacting Ni(0) with a compound (I) in the presence of a liquid diluent to give a first system comprising Ni(0) and compound (I) and then
b) reacting this first system with a compound (II) in the presence of a liquid diluent to give a system according to any of claims 1 to 10.

7. The process according to claim 6, wherein metallic nickel is used as Ni(0) in step a).

8. The process according to claim 6 or 7, wherein from 4 to 10 mol of compound (I) are used per mol of Ni(0) in step a).

9. The process according to any of claims 6 to 8, wherein the liquid diluent used in step a) is a compound of the formula (I), 3-pentenenitrile, 4-pentenenitrile, 2-methyl-3-butenenitrile, adiponitrile, methylglutaronitrile, an aromatic, an aliphatic or a mixture thereof.

10. The process according to any of claims 6 to 9, wherein the preparation of the first system in step a) is carried out in the presence of a catalyst.

11. The process according to any of claims 6 to 9, wherein the preparation of the first system in step a) is carried out in the presence of a homogeneous catalyst.

12. The process according to any of claims 6 to 9, wherein the preparation of the first system in step a) is carried out in the presence of a protic acid as homogeneous catalyst.

13. The process according to any of claims 6 to 9, wherein the preparation of the first system in step a) is carried out in the presence of hydrogen chloride as homogeneous catalyst.

14. The process according to any of claims 6 to 9, wherein the preparation of the first system in step a) is carried out in the presence of a compound of the formula (R¹X¹) (R²X²) PCl or (R¹X¹)PCl₂, where X¹, X², R¹, R² are as defined in any of claims 1 to 8, as homogeneous catalyst.

15. The process according to any of claims 6 to 14, wherein the catalyst is removed between step a) and step b).

16. The process according to any of claims 6 to 15, wherein from 1 to 4 mol of compound (II) are used per mol Ni(0) in step b).

17. The process according to any of claims 6 to 16, wherein the liquid diluent used in step b) is a compound of the formula (I), 3-pentenenitrile, 4-pentenenitrile, 2-methyl-3-butenenitrile, adiponitrile, methylglutaronitrile, an aromatic, an aliphatic or a mixture thereof.

18. The process according to any of claims 6 to 17, wherein the same liquid diluent is used in step a) and in step b).

19. A process for preparing mixtures of monoolefinic C₅-mononitriles having nonconjugated C=C and C=N bonds by hydrocyanation of a 1,3-butadiene-containing hydrocarbon mixture in the presence of a catalyst comprising at least one of the systems according to any of claims 1 to 5.

20. A process for preparing a dinitrile by hydrocyanation of a mixture of monoolefinic C₅-mononitriles having nonconjugated C=C and C=N bonds in the presence of a catalyst comprising at least one of the systems according to any of claims 1 to 5.

21. A process for preparing adiponitrile by hydrocyanation of a mixture of monoolefinic C₅-mononitriles having nonconjugated C=C and C=N bonds in the presence of a catalyst comprising at least one of the systems according to any of claims 1 to 5.

## Revendications

1. Système approprié en tant que catalyseur, contenant :
a) Ni(0) ;
b) 4 à 10 moles par mole de Ni(0) selon a) d'un composé (I) de formule
P(X¹R¹) (X²R²) (X³R³) (I) avec
X¹, X², X³ oxygène
R¹, R², R³ indépendamment les uns des autres, radicaux organiques identiques ou différents
et
c) 1 à 4 moles par mole de Ni(0) selon a) d'un composé (II) de formule 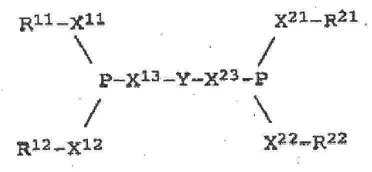 avec
X¹¹, X¹², X¹³, X²¹, X²², X²³ oxygène
R¹¹, R¹² indépendamment l'un de l'autre, radicaux organiques identiques ou différents, individuels ou pontés,
R²¹, R²² indépendamment l'un de l'autre, radicaux organiques identiques ou différents, individuels ou pontés,
Y groupe de pontage,
R¹, R², R³ indépendamment les uns des autres, groupe phényle, o-tolyle, m-tolyle ou p-tolyle.

2. Système selon la revendication 1, dans lequel R¹, R² et R³ représentent indépendamment les uns des autres un groupe phényle, o-tolyle, m-tolyle ou p-tolyle, à condition que le nombre de groupes phényle dans le composé (I) soit d'au plus 2 et que le nombre de groupes o-tolyle dans le composé (I) soit d'au plus 2.

3. Système selon la revendication 1, dans lequel en tant que composé (I), un composé de formule
(o-tolyl-O-)_{w}(m-tolyl-O-)ₓ(p-tolyl-O-)_{y}(phényl-O-)_{z}P
avec w, x, y, z un nombre naturel
avec w + x + y + z = 3 et
w, z inférieur ou égal à 2,
est utilisé.

4. Système selon les revendications 1 à 3, dans lequel Y est un pyrocatéchol, bis(phénol) ou bis(naphtol) substitué ou non substitué.

5. Système selon les revendications 1 à 4, contenant un complexe de Ni(0) de formule
Ni(0)(composé (I))ₓ(composé (II)) avec x = 1, 2.

6. Procédé de fabrication d'un système selon les revendications 1 à 5, **caractérisé en ce que** a) Ni(0) est mis en réaction avec un composé (I) en présence d'un diluant liquide pour obtenir un premier système contenant Ni(0) et le composé (I), puis b) ce premier système est mis en réaction avec un composé (II) en présence d'un diluant liquide pour obtenir un système selon les revendications 1 à 10.

7. Procédé selon la revendication 6, dans lequel du nickel métallique est utilisé à l'étape a) en tant que Ni(0).

8. Procédé selon la revendication 6 ou 7, dans lequel 4 à 10 moles de composé (I) par mole de Ni(0) sont utilisées à l'étape a).

9. Procédé selon les revendications 6 à 8, dans lequel un composé de formule (I), du 3-pentène-nitrile, du 4-pentène-nitrile, du 2-méthyl-3-butène-nitrile, de l'adipodinitrile, du méthylglutaronitrile, des composés aromatiques, des composés aliphatiques ou leurs mélanges sont utilisés à l'étape a) en tant que diluant liquide.

10. Procédé selon les revendications 6 à 9, dans lequel la fabrication du premier système à l'étape a) est réalisée en présence d'un catalyseur.

11. Procédé selon les revendications 6 à 9, dans lequel la fabrication du premier système à l'étape a) est réalisée en présence d'un catalyseur homogène.

12. Procédé selon les revendications 6 à 9, dans lequel la fabrication du premier système à l'étape a) est réalisée en présence d'un acide protonique en tant que catalyseur homogène.

13. Procédé selon les revendications 6 à 9, dans lequel la fabrication du premier système à l'étape a) est réalisée en présence de chlorure d'hydrogène en tant que catalyseur homogène.

14. Procédé selon les revendications 6 à 9, dans lequel la fabrication du premier système à l'étape a) est réalisée en présence d'un composé de formule (R¹X¹)(R²X²)PCl ou (R¹X¹)PCl₂, X¹, X², R¹, R² ayant les significations selon les revendications 1 à 8, en tant que catalyseur homogène.

15. Procédé selon les revendications 6 à 14, dans lequel le catalyseur est éliminé entre l'étape a) et l'étape b).

16. Procédé selon les revendications 6 à 15, dans lequel 1 à 4 moles du composé (II) par mole de Ni(0) sont utilisées à l'étape b).

17. Procédé selon les revendications 6 à 16, dans lequel un composé de formule (I), du 3-pentène-nitrile, du 4-pentène-nitrile, du 2-méthyl-3-butène-nitrile, de l'adipodinitrile, du méthylglutaronitrile, des composés aromatiques, des composés aliphatiques ou leurs mélanges sont utilisés à l'étape b) en tant que diluant liquide.

18. Procédé selon les revendications 6 à 17, dans lequel le même diluant liquide est utilisé à l'étape a) et à l'étape b).

19. Procédé de fabrication de mélanges de mononitriles en C₅ monooléfiniques contenant des liaisons C=C et C=N non conjuguées par hydrocyanation d'un mélange d'hydrocarbures contenant du 1,3-butadiène en présence d'un catalyseur, **caractérisé en ce que** l'hydrocyanation est réalisée en présence d'au moins un des systèmes selon les revendications 1 à 5.

20. Procédé de fabrication d'un dinitrile par hydrocyanation d'un mélange de mononitriles en C₅ monooléfiniques contenant des liaisons C=C et C=N non conjuguées en présence d'un catalyseur, **caractérisé en ce que** l'hydrocyanation est réalisée en présence d'au moins un des systèmes selon les revendications 1 à 5.

21. Procédé de fabrication d'adipodinitrile par hydrocyanation d'un mélange de mononitriles en C₅ monooléfiniques contenant des liaisons C=C et C=N non conjuguées en présence d'un catalyseur, **caractérisé en ce que** l'hydrocyanation est réalisée en présence d'au moins un des systèmes selon les revendications 1 à 5.
